(19) Europäisches Patentamt
European Patent Office
Office européen des brevets

(11) Numéro de publication: 0 200 648
B1

# (12) FASCICULE DE BREVET EUROPEEN

(45) Date de publication du fascicule du brevet: **20.04.88**

(51) Int. Cl.[4]: **C 12 P 7/64**, C 11 C 1/04, C 12 M 1/40

(21) Numéro de dépôt: **86400915.4**

(22) Date de dépôt: **25.04.86**

(54) **Procédé et appareil pour l'hydrolyse enzymatique de corps gras.**

(30) Priorité: **26.04.85 FR 8506388**

(43) Date de publication de la demande: **05.11.86 Bulletin 86/45**

(45) Mention de la délivrance du brevet: **20.04.88 Bulletin 88/16**

(84) Etats contractants désignés: **DE GB NL SE**

(56) Documents cités:
**EP - A - 0 126 416**

**SOVIET INVENTIONS ILLUSTRATED, section chemical, semaine 84/07, 28 mars 1984, no. 84/041743/07, Derwent Publications, Londres, GB**

(73) Titulaire: **SOCIETE NATIONALE ELF AQUITAINE Société anonyme dite, Tour Elf 2, Place de la Coupole La Défense 6, F-92400 Courbevoie (FR)**
Titulaire: **CECA S.A., 11 avenue Morane Saulnier, F-78141 Velizy Villacoublay (FR)**

(72) Inventeur: **Gancet, Christian, 13 Lot. de la Digue Lons, F-64140 Billere (FR)**
Inventeur: **Guignard, Claude, Maison Cadillacq Ossages, F-40290 Habas (FR)**

(74) Mandataire: **Kohn, Armand, 5 Avenue Foch, F-92380 Garches (FR)**

EP 0 200 648 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention se rapporte à un procédé et un appareil pour l'hydrolyse enzymatique de corps gras; elle s'applique plus particulièrement à la production d'acides gras à partir de leurs esters et plus spécialement des triglycérides.

La production industrielle des acides gras s'opère généralement par l'hydrolyse thermique de graisses ou huiles, en présence de la vapeur d'eau à une température assez élevée. C'est un procédé qui permet une fabrication rapide de fortes quantités d'acides désirés, mais il consomme beaucoup d'énergie et requiert des investissements coûteux; de plus, l'application d'une température élevée favorise la dégradation des matières traitées, sensible surtout en ce qui concerne les acides insaturés. Or, comme on le sait, il est possible de produire l'hydrolyse des esters gras, notamment des triglycérides, par l'action de certaines enzymes; c'est ainsi que l'action des lipases extracellulaires, secrétées par des microorganismes, est connue depuis longtemps. Dans certaines conditions de cultures, des microorganismes et en particulier certains champignons filamenteux ont la propriété d'exprimer une activité lipasique intracellulaire qui reste liée aux parois du mycélium. Le système enzyme/paroi se comporte alors comme une enzyme purifiée, immobilisée sur un support solide; le mycélium à activité enzymatique présente en fait les mêmes stabilité et commodité d'emploi que les systèmes d'enzymes immobilisées sur support, mais il est plus économique, puisqu'il est produit par voie naturelle. On a donc essayé d'appliquer un tel système à la production des acides gras. Cependant, pour obtenir des résultats viables, il a fallu employer un solvant pour dissoudre la matière grasse de départ. De telles tentatives sont décrites par BELL C, TODD J.R., BLAIN J.A., PATTERSON J D E, SHAW C.E.L., Bioteh. Bioeng (1981) 23(7) 1703-19. Ces auteurs ont utilisé le mycélium du champignon *Rhizopus arrhizus* en tant que source de lipase, et ont employé l'huile en solution dans l'éther diisopropylique. Toutefois, ces travaux n'ont conduit qu'à des rendements assez faibles, en moyenne de 20 à 50% en acides gras recherchés; d'autre part, dans ces conditions opératoires, la perte de l'activité de l'enzyme est assez sensible, d'environ 0,6 à 1% par heure. Il apparaît nettement que la proportion d'eau à introduire dans le milieu réactionnel joue un grand rôle, et les auteurs ont trouvé que l'optimum de cette concentration se situait aux environs de 0,17% en poids par rapport au volume du mélange réactionnel, et que de part et d'autre de cette valeur la conversion de la matière grasse baisse fortement. Il résulte de cet art antérieur que la quantité d'eau tolérée doit même être inférieure à la proportion stœchiométrique correspondant aux esters traités. En définitive, bien que le principe décrit paraisse intéressant, sa réalisation industrielle ne pourrait pas être envisagée à cause des inconvénients susmentionnés.

La présente invention apporte un perfectionnement sensible au principe de l'hydrolyse des matières grasses en présence d'un mycélium porteur de lipase, au sein d'un solvant organique, par des modifications inattendues qui permettent l'obtention de résultats vraiment industriels. En effet, perfectionné suivant l'invention, le procédé précité est susceptible de fournir des acides gras avec des rendements dépassant 80% et même 90% sur la matière grasse mise en œuvre, alors que la désactivation de l'enzyme est extrêmement lente, ne dépassant généralement pas 0,01% par heure.

Bien entendu, le procédé de l'invention, comme tous ceux qui utilisent les enzymes, bénéficie des conditions opératoires très douces, puisqu'on opère en général à des températures ne dépassant pas 50° C et, le plus souvent, entre 15 et 35° C. Cela évite toute dégradation des acides gras formés et fournit par conséquent un produit plus pur que la technique industrielle classique.

Le procédé suivant l'invention, qui consiste à hydrolyser une matière grasse dissoute dans un solvant organique, additionné d'une faible quantité d'eau, en présence du mycélium porteur de lipase, est caractérisé en ce que le milieu réactionnel, comprenant l'eau, est rendu homogène par l'adjonction d'un agent susceptible d'y dissoudre ou microémulsionner l'eau nécessaire à l'hydrolyse.

Dans une première forme d'exécution, l'agent d'homogénéisation est constitué par un solvant tiers, capable de former une phase unique entre le solvant contenant la matière grasse et l'eau. De tels solvants tiers peuvent être choisis par exemple parmi des cétones, des amides, des solvants azotés cycliques, etc. Ainsi, peut-on employer des liquides tels que: acétone, méthyl-éthylcétone, diéthylcétone, acétophénone, glycérol-formal, diméthyl-acétamide, N-méthyl-pyrrolidone, etc. Ce solvant tiers doit être naturellement présent en une proportion adéquate pour produire la dissolution de l'eau dans la solution du corps gras employé dans le solvant organique; cette proportion dépend de la nature des autres matières en présence, mais elle est généralement de l'ordre de 5 à 30% du total.

En tant qu'exemples non limitatifs de cette forme d'exécution, voici les proportions particulières des constituants du mélange réactionnel:

| | |
|---|---|
| Corps gras | 10 à 30% en poids |
| Acétone | 15 à 20% en poids |
| Eau | 1 à 3% en poids |
| Solvant organique du corps gras | 58 à 74% en poids |

Un tel mélange est parfaitement limpide à la température d'opération de 30° C, et ne donne lieu à aucune démixtion au cours de la réaction.

Dans la variante de l'invention, qui consiste à microémulsionner l'eau dans la solution de la matière grasse, un solvant tiers peut ne pas être nécessaire, lorsque le solvant organique de la matière grasse, lui-même, peut servir de co-tensioactif à l'émulsifiant que l'on ajoute au milieu, dans cette variante. Ici il est important d'incorporer, au mélange réactionnel, un agent tensioactif convenable. La préparation de microémulsions de l'eau dans des solvants hydrophobes étant connue dans

l'art, il n'y a pas lieu d'en donner ici des détails. On notera cependant que des agents tensioactifs tels que sulfates d'alcools gras, sulfosuccinates, esters oxyéthylénés de sorbiton, alcools, acides ou huiles oxyéthylénés, esters de saccharose, polyglycols, phosphates d'alkyle lourds, etc., conviennent particulièrement bien à la réalisation de l'invention.

Comme on le sait, dans une microémulsion de l'eau au sein d'un solvant hydrophobe, l'eau se trouve généralement à l'intérieur des micelles inverses, constituées par un composé tensioactif accompagné d'un co-tensioactif. Ce dernier peut être le solvant tiers indiqué plus haut, ou bien le solvant organique lui-même, du corps gras.

Cette forme d'exécution de l'invention présente, entre autres, l'avantage de permettre d'élever jusqu'à environ 30% la teneur en corps gras du solvant organique utilisé, sans que le tout cesse de présenter l'aspect d'une véritable solution.

A titre d'exemple non limitatif, la composition d'un milieu, soumise à l'action enzymatique d'un mycélium, peut se composer en poids de

| | |
|---|---|
| Corps gras | 10 à 45% |
| Tensioactif (plus éventuellement co-tensioactif) | 1 à 5% |
| Eau | 1 à 3% |
| Solvant organique | 47 à 88% |

Une autre particularité tout à fait nouvelle, de l'invention, réside en ce que l'eau, nécessaire à l'hydrolyse de la matière grasse, est introduite à différents moments de l'avancement de la réaction. On compense ainsi la quantité d'eau consommée par la réaction qui peut être schématisée de la façon suivante dans le cas des triglycérides correspondant à des acides gras des groupes $R^1$, $R^2$ et $R^3$:

$$\begin{bmatrix} OCO\ R^1 \\ OCO\ R^2 \\ OCO\ R^3 \end{bmatrix} + \xrightarrow{3H_2O} \begin{bmatrix} OH \\ OH \\ OH \end{bmatrix} \begin{matrix} R^1COOH \\ +\ R^2COOH \\ R^3COOH \end{matrix}$$

Cette eau peut être introduite en continu ou en discontinu, selon la technique adoptée pour l'exécution du procédé.

La caractéristique de l'invention, qui consiste à dissoudre ou microémulsionner l'eau dans le milieu réactionnel, rend possible l'utilisation d'un certain excès en $H_2O$ par rapport à la proportion stœchiométrique; cela s'accompagne d'un accroissement très sensible du rendement en acides gras obtenus. Alors que dans la technique antérieure, citée plus haut, la quantité d'eau permise n'atteint pas celle qui serait nécessaire au point de vue stœchiométrique, le procédé suivant l'invention utilise des quantités de $H_2O$ nettement supérieure à la théorie; cette quantité est généralement comprise entre 1 à 2 fois la quantité théorique, et, de préférence, 1,1 à 1,6.

Dans le procédé de l'art antérieur cité plus haut, le choix des solvants organiques pour la matière grasse de départ est fortement limité, parce que la plupart des solvants, compatibles avec les enzymes utilisées, ne permettent pas la mise en solution de la quantité d'eau nécessaire à la réaction. Par contre, le perfectionnement suivant l'invention élargit grandement le domaine des solvants utilisables, puisque l'eau y est dissoute à l'aide d'un solvant tiers ou de la microémulsion. C'est ainsi qu'à titre d'exemple, les solvants de la liste qui suit peuvent être utilisés dans le procédé de l'invention.

| *Solvant* | *Activité relative (%)* |
|---|---|
| Di-éthyl-éther (DEE) | 125 |
| Di-isopropyl-éther (DIPE) | 100 |
| Méthyl-t-butyl-éther (MTBE) | 94,6 |
| Di-isobutyl-éther (DIBE) | 89,2 |
| Di-n-butyl-éther | 78,4 |
| Di-méthoxy-propane (DMP) | 75,0 |
| Phényl éthyl éther (Phénétole) | 35,1 |
| Phényl éthyl éther (Anisole) | 27,0 |
| 1,2-diéthoxy-éthane | 32,4 |
| 1,2-diméthoxy-éthane | 24,3 |
| Diméthyl éther du diéthylène glycol (diglyme) | 24,0 |
| Dioxane | 18,9 |
| Diméthylformamide (DMF) | 0 |
| Diméthyl sulfoxyde (DMSO) | 0 |

Les activités relatives pour ces différents solvants ont été déterminées par l'hydrolyse de l'huile d'olive dans les conditions suivantes:

| | |
|---|---|
| huile d'olive % en poids | 10 |
| eau | 0,5 |
| acétone | 5 |
| solvant | 84,5 |

0,1 g de mycélium par gramme d'huile, réaction discontinue à 30° C pendant 60 minutes.

Le diéthyl-éther ne peut pas être employé pratiquement à cause de sa forte volatilité. Parmi les autres solvants, les demandeurs ont sélectionné le méthyl-t-butyl-éther (MTBE) comme solvant particulièrement intéressant, parce qu'il est beaucoup moins toxique que le di-isopropyl-éther de l'art antérieur et qu'il présente, par rapport à tous les autres solvants viables, la tendance la plus faible à former des dérivés peroxydés dangereux. L'application de ce solvant fait donc partie de la nouveauté de l'invention.

Etant donné que la culture des microorganismes, la récolte et traitement ultérieur de mycélium obtenu, pour servir d'enzyme immobilisée, sont connus, il n'y a pas lieu de les décrire ici. On doit cependant noter que l'invention s'applique à des mycélia provenant de différents microorganismes, dont on cite ci-après quelques-uns, seulement à titre d'exemples non limitatifs.

Les *Rhizopus stolonifer, arrhizus, delemar* et *japonicus*; *Geotrichum candidum*; *Mucor javanicus, hiemalis* et *miehei*; *Aspergillus oryzae* et *niger*.

L'invention comprend également un appareillage pour la réalisation du procédé décrit plus haut. L'installation comprend au moins une capacité de stockage du mélange réactionnel, au moins une colonne garnie de matières de remplissage appropriées supportant l'enzyme et des moyens de transport du mélange réactionnel du stockage à la colonne. Las nouveauté de l'appareil suivant l'invention réside en ce que celui-ci est également muni de moyens d'introduction de l'eau à différents stades d'avancement du mélange réactionnel.

A titre d'exemple, on décrit, ci-après, une installation qui a donné les bons résultats décrits dans les exemples qui suivent.

Le dessin unique, annexé, représente schématiquement une installation suivant l'invention, comportant trois colonnes à réaction enzymatique. Bien entendu, le nombre de colonnes ou réacteurs peut varier selon les besoins.

La cuve 1 constitue la capacité nécessaire au mélange et au stockage des matières de départ. Elle est munie d'un agitateur 2 et de quatre admissions de liquides: repère 3 pour le solvant du corps gras, 4 pour ce dernier lui-même, 5 pour le solvant tiers, ou/et pour un agent tensioactif, et 6 pour l'eau. Le mélange, homogénéisé dans 1, est repris par la pompe P1 pour être envoyé au bas de la colonne de réaction 7. Celle-ci contient le mycélium enzymatique. De préférence, celui-ci est mélangé avec des grains d'un support minéral, en particulier silice, alumine, silicoaluminate, verre, etc.

Le dessin représente le cas où le mélange réactionnel chemine de bas en haut, dans la colonne 7, mais il est bien entendu que l'invention peut être également réalisée avec la disposition connue de circulation de haut en bas. Elle peut d'ailleurs être mise en œuvre avec un lit fluidisé.

La colonne 7 est entourée d'une chemise 8 pour la circulation d'un fluide thermostatique.

Le liquide qui sort de la colonne 7 par la canalisation 9, en vue de son passage dans une seconde colonne 12, est d'abord reçu dans un mélangeur intermédiaire 11, où il est agité avec une portion d'eau d'ajustement, introduite par 10. La proportion d'eau, ainsi rajoutée, est telle qu'il y ait toujours, dans le milieu réactionnel, suffisamment d'eau, notamment 1 à 2 fois la quantité stœchiométrique correspondant à la matière grasse présente en une zone de réaction donnée.

Ainsi, le mélange 11 intermédiaire, entre les colonnes 7 et 12, qui n'existe pas dans les installations classiques d'hydrolyse enzymatique, constitue-t-il un élément nouveau, caractéristique de l'appareillage suivant l'invention.

Du mélangeur 11, par la pompe P2, le mélange à teneur en eau ajustée est transféré dans la colonne 12 entourée d'un chemisage thermostatique 13. L'hydrolysat passe ensuite, par une canalisation 14, dans un second mélangeur intermédiaire 16, alimenté en eau d'appoint par 15, et fonctionnant comme le précédent (11). De 16 le mélange est envoyé, au moyen de la pompe P3, dans une 3$^{e}$ colonne (17), et ainsi de suite s'il le faut.

L'invention est illustrée par les exemples non limitatifs suivants.

*Exemple 1:*

L'hydrolyse du suif est effectuée au moyen des lipases d'un mycelium de *Rhizopus arrhizus* (ATCC 24563), dans un appareil de laboratoire constitué comme décrit plus haut avec référence au dessin.

On fait passer un mélange initial, homogène, ayant la composition suivante:

| | |
|---|---|
| suif | 20 (corps gras) |
| méthyl t.butyl éther (MTBE) | 58 (solvant organique) |
| eau | 2 (environ 1,6 fois stœchiométrique) |
| acétone | 20 (solvant tiers) |

successivement par les trois colonnes, à 30° C.

Chaque colonne contient 5,5 g de mycélium en poudre mélangée avec 10 ml de silice en grains de 0,05 mm de moyenne, en tout un volume de 35 ml.

Le volume mort par colonne est d'environ 15 ml pour un débit de l'ordre de 12 ml/h, ce qui correspond à un temps de séjour du mélange liquide de 75 minutes environ dans chaque colonne. Le taux de conversion du suif, après la première colonne (repère 7 sur le dessin), atteint 60%. On ajuste alors la teneur en eau du mélange traité, par l'addition de 0,8% d'eau dans le premier mélangeur intermédiaire (11 sur le dessin). A la sortie de la 2$^{e}$ colonne (12) la conversion du suif est de 83% et l'on ajoute 0,7% d'eau dans le mélangeur intermédiaire (16) qui la suit.

A la suite de la troisième colonne (17) la conversion du suif ressort à 91%.

Après l'évaporation des solvants on recueille 71,8 g d'hydrolysat ayant la composition suivante en acides gras (en poids):

| | |
|---|---|
| myristique ($C_{14}$) | 2,6 |
| palmitique ($C_{16}$) | 30,4 |
| palmitoléique ($C_{16}$:1) | 3,3 |
| stéarique ($C_{18}$) | 21,3 |
| oléique ($C_{18}$:1) } linoléique ($C_{18}$:2) } | 42,0 |

déterminée par chromatographie en phase gazeuse des dérivés triméthyl-silylés d'échantillons d'hydrolysats.

*Exemple 2:*

Comparatif avec la technique antérieure.

On opère comme dans l'exemple 1, dans le même appareil, mais sans adjonction d'acétone, donc sans tiers solvant et sans ajustement de l'eau dans les mélangeurs intermédiaires.

La composition des 100 g de mélange initial est ici:

| | |
|---|---|
| suif | 20 g |
| méthyl t.butyl éther | 79 g |
| eau | 1 g (limite de solubilité) |

La conversion du suif après la 3$^{e}$ colonne est alors seulement de 27%.

Cela prouve le remarquable avantage du mode opératoire de l'exemple 1.

*Exemple 3:*

Dans une hydrolyse, menée avec le tiers solvant (acétone) comme dans l'exemple 1, mais sans addition d'eau dans les mélangeurs intermédiaires (11 et 16), la conversion après la 3$^{e}$ colonne (17) est de 65% donc moindre que dans l'exemple 1, tout en restant encore bien supérieure à celle de la technique antérieure, illustrée par l'exemple 2. La

teneur en eau au départ étant de 10% par rapport au suif, soit environ 1,6 fois la quantité théorique, nécessaire, elle n'a pas pu baisser au-dessous de la valeur stœchiométrique au cours de la réaction. L'amélioration de la conversion du fait de l'ajustement de la proportion d'eau entre colonnes successives est donc un résultat tout à fait imprévu, s'ajoutant à celui qu'apporte l'emploi d'un solvant tiers.

*Exemple 4:*

L'appareil et les opérations sont identiques à ceux de l'exemple 1, mais appliqués à de l'huile de colza en tant que substrat.

Le mélange de départ se composait de:

(pour 100 g)

20 g d'huile de colza
63,5 g MTBE
1,5 g d'eau
15 g d'acétone.

Le poids d'eau, introduite au départ, représente ainsi 7,5% de celui de l'huile, soit environ 1,25 fois la proportion théoriquement nécessaire.

On a alors:

| | *Conversion* % | *g $H_2O$ ajoutée* |
|---|---|---|
| après la 1re colonne | 55 | 1,1 |
| après la 2[e] colonne | 63 | 0,7 |
| après la 3[e] colonne | 70 | — |

La masse d'hydrolysat, recueilli après l'élimination des solvants, est de 95,5 g et sa composition en acides gras est la suivante:

| | |
|---|---|
| palmitique | 7,8 |
| stéarique | 2,8 |
| oléique, linoléique, linolénique | 85,3 |
| $C_{20}$ et $C_{22}$ | 4,1 |

*Exemple 5:*

Hydrolyse du suif en microémulsion.

Dans les mêmes conditions qu'à l'exemple 1, on effectue l'hydrolyse du suif en solution dans du méthyl tert-butyl éther (MTBE), l'eau étant microémulsionnée dans cette solution par l'addition d'un agent tensioactif.

Le mélange de départ est constitué par:

30,6 g de suif
66 g de solvant MTBE
2,1 g d'eau (soit 6,8% par rapport au suif)
1,3 g de dioctyl-sulfo-succinate de Na (tensioactif)

Ce mélange est bien homogène tout au long de son passage dans les colonnes de réaction.

Après la 1re colonne, la conversion est de 60,8%, et l'on ajoute 1,8 g d'eau, ce qui fait porter la proportion d'eau par rapport au suif restant à environ 10%.

A la sortie de la seconde colonne la conversion s'est élevée à 83,5%.

On recueille 120 g d'hydrolysat dont les acides gras se répartissent comme suit:

| | |
|---|---|
| myristique | 3,3 |
| palmitique | 27,5 |
| palmitoléique | 4,1 |
| stéarique | 20,0 |
| oléique, linoléique | 45,2 |

On voit qu'en microémulsion les résultats sont pratiquement les mêmes qu'avec le tiers solvant de l'exemple 1, mais avec l'avantage supplémentaire de permettre l'utilisation d'une plus forte charge de corps gras au départ, notamment 30,6 au lieu de 20.

*Exemple 6:*

Hydrolyse sélective des acides gras insaturés.

Le catalyseur utilisé est un mycélium de Geotrichum Candidum préparé de façon identique à celui de Rhizopus.

Le substrat utilisé est le même que celui de l'exemple 1: il est envoyé sur une seule colonne contenant 5,5 g de mycélium mélangé à 10 ml de silice.

Un temps de séjour de l'ordre de 75 min, semblable aux exemples précédents, permet d'obtenir un taux de conversion de 18%.

Si l'on recueille cet hydrolysat, la composition suivante en acide gras libre est observée:

| | |
|---|---|
| acide myristique | 2,3% |
| acide palmitique | 11,9% |
| acide palmitoléique | 2,3% |
| acide oléique et linoléique | 76,1% |
| acide stéarique | 7,4% |

La teneur totale en acides gras insaturés est donc de 78,4% contre 45,3% dans l'exemple 1 où l'on utilisait une lipase de Rhizopus.

Il y a donc hydrolyse sélective des acides gras insaturés.

**Revendications**

1. Procédé d'hydrolyse enzymatique d'une matière grasse en vue de la libération des acides gras qu'elle contient, par contact d'une solution de cette matière, dans un solvant organique, avec de l'eau, en présence d'un mycélium porteur de lipase, caractérisé en ce que cette eau est homogénéisée avec le solvant organique à l'aide d'un tiers solvant ou d'un agent tensioactif.

2. Procédé suivant la revendication 1, caractérisé en ce que le tiers solvant est une cétone, ou un amide.

3. Procédé suivant la revendication 2, caractérisé en ce que le tiers solvant est l'acétone.

4. Procédé suivant une des revendications précédentes, caractérisé en ce que la proportion de tiers solvant est d'environ 5 à 30% du mélange soumis à l'hydrolyse.

5. Procédé suivant la revendication 1, caractérisé en ce que l'eau est microémulsionnée avec la solution du corps gras dans le solvant organique,

par l'adjonction d'environ 1 à 5% d'un agent tensioactif.

6. Procédé suivant la revendication 5, caractérisé en ce que le tensioactif est accompagné d'un co-tensioactif, lorsque le solvant organique du corps gras ne joue pas lui-même le rôle de ce dernier.

7. Procédé suivant la revendication 4, caractérisé en ce que le mélange à traiter contient 10 à 30% de corps gras.

8. Procédé suivant la revendication 5 ou 6, caractérisé en ce que le mélange à traiter contient 10 à 45% de corps gras.

9. Procédé suivant une des revendications précédentes, caractérisé en ce que la teneur en eau du mélange à traiter est supérieure à la proportion stoechiométrique par rapport aux corps gras, et elle est en particulier 1 à 2 fois la quantité théorique.

10. Procédé suivant une des revendications précédentes, caractérisé en ce que le solvant organique pour la matière grasse est le méthyl-tert.butyl éther.

11. Procédé suivant une des revendications précédentes, caractérisé en ce qu'une addition d'eau est effectuée à différents stades de la réaction, en continu ou en discontinu.

12. Appareillage pour la réalisation du procédé suivant une des revendications 1 à 11, comprenant un premier mélangeur (1) et une série de colonnes successives (7, 12, 16) renfermant du mycélium porteur de lipase, ainsi que des moyens (P1, P2, P3) pour faire circuler le mélange liquide à traiter dans les colonnes, caractérisé en ce qu'entre chaque colonne et la colonne suivante est branché un mélangeur intermédiaire (11, 16) équipé pour l'addition de l'eau au mélange circulant.

## Patentansprüche

1. Enzymatisches Hydrolyseverfahren für ein Fettmaterial zur Freisetzung von darin enthaltenen Fettsäuren durch Kontakt einer Lösung dieses Materials mit Wasser in einem organischen Lösungsmittel in Gegenwart eines Myzels, das Lipase aufweist, dadurch gekennzeichnet, dass das Wasser mit dem organischen Lösungsmittel mittels eines dritten Lösungsmittels oder eines oberflächenaktiven Mittels homogenisiert wird.

2. Verfahren nach Anspruch 1, dadurch gekennzeichnet, dass es sich beim dritten Lösungsmittel um ein Keton oder ein Amid handelt.

3. Verfahren nach Anspruch 2, dadurch gekennzeichnet, dass es sich beim dritten Lösungsmittel um Aceton handelt.

4. Verfahren nach einem der vorstehenden Ansprüche, dadurch gekennzeichnet, dass der Anteil des dritten Lösungsmittels etwa 5 bis 30% des der Hydrolyse unterworfenen Gemisches beträgt.

5. Verfahren nach Anspruch 1, dadurch gekennzeichnet, dass das Wasser mit der Lösung der Fettmasse im organischen Lösungsmittel durch Zugabe von etwa 1 bis 5% eines oberflächenaktiven Mittels mikroemulgiert wird.

6. Verfahren nach Anspruch 5, dadurch gekennzeichnet, dass das oberflächenaktive Mittel von einem Co-(oberflächenaktiven Mittel) begleitet ist, wenn das organische Lösungsmittel für die Fettmasse nicht selbst die Rolle des letzteren übernimmt.

7. Verfahren nach Anspruch 4, dadurch gekennzeichnet, dass das zu behandelnde Gemisch 10 bis 30% Fettmasse enthält.

8. Verfahren nach Anspruch 5 oder 6, dadurch gekennzeichnet, dass das zu behandelnde Gemisch 10 bis 45% Fettmasse enthält.

9. Verfahren nach einem der vorstehenden Ansprüche, dadurch gekennzeichnet, dass der Wassergehalt des zu behandelnden Gemisches grösser als der stöchiometrische Anteil in bezug auf die Fettmasse ist und insbesondere das 1 - bis 2-fache der theoretischen Menge beträgt.

10. Verfahren nach einem der vorstehenden Ansprüche, dadurch gekennzeichnet, dass es sich beim organischen Lösungsmittel für die Fettmasse um Methyl-tert.-butylether handelt.

11. Verfahren nach einem der vorstehenden Ansprüche, dadurch gekennzeichnet, dass eine Zugabe von Wasser in verschiedenen Reaktionsstadien kontinuierlich oder diskontinuierlich vorgenommen wird.

12. Vorrichtung zur Durchführung des Verfahrens nach einem der Ansprüche 1 bis 11, umfassend eine erste Mischeinrichtung (1) und eine Reihe von aufeinanderfolgenden Kolonnen (7, 12, 16), die Myzel mit einem Gehalt an Lipase enthalten, sowie Vorrichtungen (P1, P2, P3) zur Zirkulation des in den Kolonnen zu behandelnden flüssigen Gemisches, dadurch gekennzeichnet, dass zwischen jeweils einer Kolonne und der anschliessenden Kolonne eine Zwischenmischeinrichtung (11, 16) angeordnet ist, die für Zugabe von Wasser zum zirkulierenden Gemisch ausgerüstet ist.

## Claims

1. Process of enzymatic hydrolysis of a fatty substance for the purpose of liberation of the fatty acids which it contains, by contact of a solution of this substance in an organic solvent with water in the presence of a mycelium carrying lipase, characterised in that the water is homogenized with the organic solvent by means of a third solvent or a surface-active agent.

2. Process according to claim 1, characterised in that the third solvent is a ketone or an amide.

3. Process according to claim 2, characterised in that the third solvent is acetone.

4. Process according to any of the preceding claims, characterised in that the proportion of the third solvent is about 5 to 30% of the mixture subjected to hydrolysis.

5. Process according to claim 1, characterised in that the water is micro-emulsified with the solution of the fatty substance in the organic solvent, by the addition of about 1 to 5% of a surface-active agent.

6. Process according to claim 5, characterised

in that the surfactant is accompanied by a co-surfactant, when the organic solvent of the fatty substance does not itself play the latter role.

7. Process according to claim 4, characterised in that the mixture to be treated contains 10 to 30% of the fatty substance.

8. Process according to claim 5 or 6, characterised in that the mixture to be treated contains 10 to 45% of the fatty substance.

9. Process according to any of the preceding claims, characterised in that the water content of the mixture to be treated is greater than the stoichiometric proportion with respect to the fatty substance and it is in particular 1 to 2 times the theoretical quantity.

10. Process according to any of the preceding claims, characterised in that the organic solvent for the fatty substance is methyl-tert.butyl ether.

11. Process according to any of the preceding claims, characterised in that an addition of water is effected at various stages in the reaction, continuously or discontinuously.

12. Apparatus for carrying out the process according to any of claims 1 to 11, comprising a first mixer (1) and a series of successive columns (7, 12, 16) containing the mycelium carrying the lipase, as well as means (P1, P2, P3) for circulating the liquid mixture to be treated in the columns, characterised in that between each column and the following column is connected an intermediate mixer (11, 16) equipped for the addition of water to the circulating mixture.

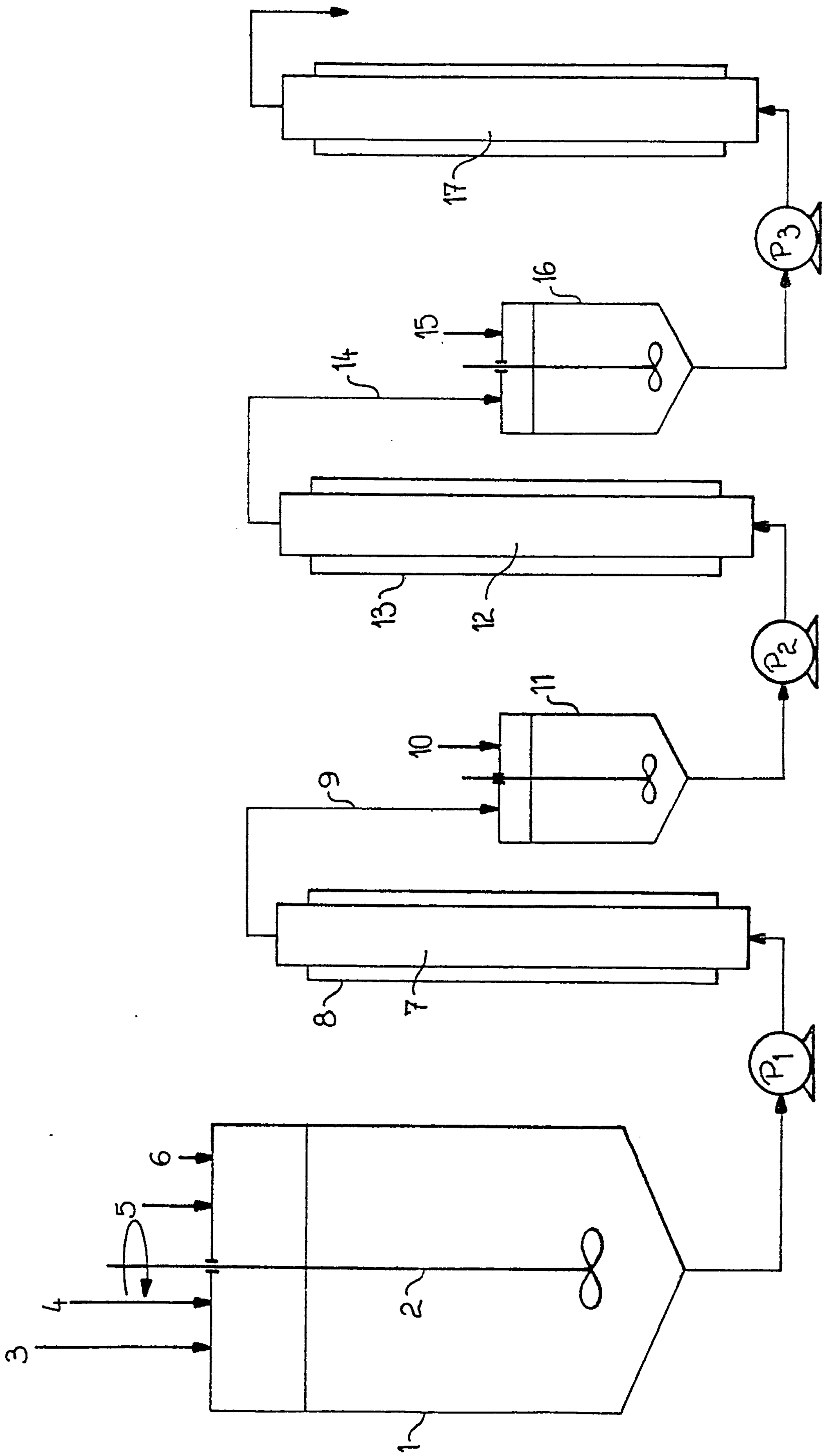
1
2
3
4
5
6
7
8
9
10
11
12
13
14
15
16
17
P1
P2
P3